(19) Europäisches Patentamt / European Patent Office / Office européen des brevets

(11) **EP 2 899 499 A1**

(12) **EUROPEAN PATENT APPLICATION**

(43) Date of publication:
**29.07.2015 Bulletin 2015/31**

(51) Int Cl.:
***G01B 15/02*** *(2006.01)* ***G01B 11/06*** *(2006.01)*

(21) Application number: **14152844.8**

(22) Date of filing: **28.01.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **ABB Technology AG**
**8050 Zürich (CH)**

(72) Inventors:
- **Van Mechelen, Jacobus Lodevicus Martinus**
  **8104 Regensdorf (CH)**
- **Merbold, Hannes**
  **8037 Zürich (CH)**

(74) Representative: **Savela, Reino Aleksi**
**ABB AB**
**Intellectual Property**
**Ingenjör Bååths Gata 11**
**721 83 Västerås (SE)**

(54) **Sensor system for characterizing a coating such as a paint film by THz radiation**

(57) A sensor system for characterizing a coating 4, such as a paint film, of a coated body 2 in a non-contact manner by use of THz radiation is provided. The sensor system comprises a THz system 1, a processing unit 30 and a positioning system 40. The THz system 1 comprises a light source 50 for generating a source light radiation; a flexible first radiation guide cable 51 for transmitting the source light radiation; a THz emitter 10 comprising a THz radiation generator 12 coupled to the light source 50 via the flexible first radiation guide cable 51 for receiving the source light radiation from the light source 50 and adapted for generating outgoing THz radiation 60 from the source light radiation, and a THz optical system 14 for directing the outgoing THz radiation towards the coated body 2; and a THz detector 20 for detecting incoming THz radiation 70 having interacted with the coating 4. The positioning system 40 comprises a movable unit 42 carrying the THz emitter 10 so that by moving the movable unit 42 the THz emitter 10 is positioned relative to the coated body 2. The light source 50 is arranged outside of the movable unit 42 so that when the movable unit 42 is moved, the THz emitter 10 is moved relative to the light source 50 while the THz emitter 10 keeps being coupled to light source 50 via the flexible first radiation guide cable 51. The processing unit 30 is operationally coupled to the THz detector 20 for receiving and processing a detected response signal representing the detected THz radiation.

**FIG. 2**

**FIG. 6**

**Description**

Aspects of the invention relate to a sensor system for characterizing a coating of a coated body, in particular of a freshly applied paint film of an automobile or the like. The sensor system has a THz emitter carried by a movable unit. Other aspects of the invention relate to a painting facility having the sensor system.

Technical background:

Automobile bodies are covered by multiple layers of paint, collectively referred to as a paint film, to protect against oxygen and other harmful substances in the environment, and for aesthetics. The painting process at automobile manufacturers is mainly done in paint process lines, in which the painting of the often metallic frames is performed by robots. The line has many aligned compartments and a central transportation system by which the automobile bodies are moved in-line from one section to the next one. After the painting process of a specific layer is finished, the frame enters a flash-off area where the solvents have the time to evaporate at room temperature, followed by a curing stage inside a furnace at elevated temperatures. Quality control is presently performed after each furnace or after the final furnace in the line.

For this purpose, prior art techniques such as ultrasonic and magnetic sensing have been developed for determining the thickness of paint layers. However, these techniques only work in contact mode, which is generally undesired. For example, patent US 6484121 B1 discloses a quality control system for the inspection of painted automobile bodies based on acoustic sensing.

In addition, recently methods based on THz radiation have been proposed. For example, JP 2004028618 A and EP 2213977 A1 describe respective methods for determining the thickness of a paint film using THz radiation.

On average 28% of the automobile bodies do not pass the quality control and have to be reworked, either by paint robots or manually. This step is a major complication for the production line. An automobile frame spends one third of the total production time in the paint line, which explains that painting of the automobile body is among the most expensive steps of the automobile manufacturing process. The cost and complexity of the reworking, in particular, thus adds significantly to the total manufacturing cost. It is therefore a longstanding desire to keep the rate of reworking low and to reduce the product line complexity at this step.

Summary of the invention

In view of the above, a sensor system according to claim 1 and a painting facility according to claim 12 are provided.

According to a first aspect, a sensor system for characterizing a coating, such as a paint film, of a coated body in a non-contact manner by use of THz radiation is provided. The sensor system comprises a THz system, a processing unit and a positioning system. The THz system comprises a light source (e.g. laser source) for generating a source light radiation (e.g. source laser radiation); a flexible first radiation guide cable for transmitting the source laser radiation; a THz emitter comprising a THz radiation generator coupled to the laser source via the flexible first radiation guide cable for receiving the source laser radiation from the laser source and adapted for generating outgoing THz radiation from the source laser radiation, and a THz optical system for directing the outgoing THz radiation towards the coated body; and a THz detector for detecting incoming THz radiation having interacted with the coating. The positioning system comprises a movable unit carrying the THz emitter so that by moving the movable unit the THz emitter is positioned relative to the coated body. The laser source is arranged outside of the movable unit so that when the movable unit is moved, the THz emitter is moved relative to the laser source while the THz emitter keeps being coupled to laser source via the flexible first radiation guide cable. The processing unit is operationally coupled to the THz detector for receiving and processing a detected response signal representing the detected THz radiation.

According to a second aspect, a painting facility for painting a body is provided, the painting facility comprises a painting unit for applying a paint layer to the body; and the sensor system according to any aspect or embodiment described herein. The sensor system is adapted for characterizing the coating including the applied paint layer in a non-contact manner by use of the THz radiation.

The sensor assembly according to embodiments of the invention allows for obtaining an accurate and meaningful set of paint parameter(s), in particular a reliable thickness of a paint layer. This is achieved by making use of a large amount of information from the detected THz radiation response of the painted body, by fitting the predicted response of a physical model to the detected THz response signal.

Thereby, embodiments of the invention open ways to perform industrial quality control of coatings such as paint films in a short amount of time, and potentially even before the paint has dried. This is enabled by the THz optical system allowing a non-contact measurement, and by the laser source being arranged outside the movable unit and thereby potentially away from any inflammable or explosive solvents evaporating from the paint during the drying process.

The fast quality control, in turn, allows removing products having failed the quality control more quickly from the production line and re-directing them to a process route in which the faults are corrected, thereby optimizing the process route. In addition, corrections before drying of the paint and generally a shortening of the process time and increase of the product yield are potentially enabled.

Further advantages, features, aspects and details that can be combined with embodiments described herein are evident from the dependent claims, the description and the drawings.

Brief description of the Figures:

The details will be described in the following with reference to the figures, wherein

Fig. 1    is a schematic view illustrating the operation of a sensor system according to an embodiment of the invention;

Fig. 2    is a schematic side view of a sensor system according to an embodiment of the invention;

Fig. 3    is a schematic view showing the THz system and related components of a sensor system according to an embodiment of the invention;

Fig. 4    is a schematic view showing the positioning system and related components of a sensor system according to an embodiment of the invention;

Fig. 5    is a schematic view illustrating possible further details and variants of the sensor system of Fig. 1;

Fig. 6    is a schematic view illustrating the interaction of THz radiation emitted by a sensor system according to an embodiment of the invention with a coated body;

Fig. 7    is a block diagram illustrating a method of characterizing a painted body used in an embodiment of the invention; and

Fig. 8    is a schematic view of a painting facility according to an embodiment of the invention.

Detailed description of aspects of the invention

In the following, some more aspects of the invention are described. Unless explicitly stated otherwise, the aspects are independent of each other and can be combined in any manner. For example, any aspect or embodiment described in this document can be combined with any other aspect or embodiment.

First, some general aspects of the sensor system are described. According to an aspect, the sensor system is adapted for a non-contact measurement, i.e. without any sensor component requiring direct physical contact with the coated body. This does not exclude a holder holding the coated body, or any further sensor component other than the THz emitter and receiver having contact with the coated body, in particular with an uncoated portion of the coated body or a portion being coated differently than the sensed coating.

Next, some aspects relating to the coated body are described in more detail. According to one aspect, the coating is multi-layered having at least a first and a second coating layer. The layers are arranged, in thickness direction of the coated body, on top of one another. According to an aspect, the total number of coating layers is eight or less. According to an aspect, the coating is less than 200 $\mu$m thick.

According to a further aspect, the coating is a paint film. The paint film may comprise at least one of the following layers (a)-(d): (a) an e-coat layer; (b) a primer layer; (c) a base coat layer; and (d) a clear coat layer. According to a further aspect, the coated body is one of an automobile component, a train component, an aircraft component, and a wind turbine component. According to a further aspect, the coated body comprises at least one of a ferrous metal, a non-ferrous metal, and a fiber composite material as a substrate on which the coating layer is applied (optionally with other coating layers in between).

Next, some aspects relating to the positioning system and the movable unit are described in more detail. According to an aspect, the movable unit is movable with at least one (lateral) degree of freedom. According to an aspect, the THz emitter and THz detector are configured for moving in at least two dimensions (two lateral degrees of freedom) along a surface of the coated body, thereby generating a position-dependent thickness map of the coating. For example, this aspect may be useful for mapping a surface area of the coated body. According to an aspect, the positioning system is adapted for moving the movable unit with at least 2 degrees of freedom, preferably with at least 3 degrees of freedom (e.g. 2 or 3 lateral degrees of freedom), and most preferably with 6 degrees of freedom, i.e. three lateral and three rotational degrees of freedom.

According to an aspect, the movable unit carries not only the THz emitter but also the THz detector, so that by moving the movable unit the THz emitter and the THz detector are positioned relative to the coated body. This does not exclude that the THz detector and/or emitter are themselves movable with respect to the movable unit 42. For example, the THz detector and/or emitter may be carried, individually or collectively, via an actuator system that enables relative movement of the THz detector and/or emitter.

According to an aspect, the positioning system further has a surface detecting unit (e.g. a movable optical or acoustic distance sensor) configured for detecting a surface orientation and/or surface curvature of a portion of the coated body facing the THz emitter and/or the THz detector. In this case, an actuation controller operationally coupled to the actuator system (46) and to the surface detecting unit may be configured for controlling the actuator system to move the THz emitter relative to the THz detector in response to the detected surface orientation and/or surface curvature. In particular, the controlling may be such that the THz detector is placed and oriented for receiving radiation of the THz emitter specularly reflected by the surface.

Next, some aspects relating to the THz system are described in more detail. According to an aspect, the radiation guide cable is a flexible fiber-optics cable. The radiation guide cable may be at least 3m, preferably at least 5m long. This allows the movable unit to have sufficient room for movement. In addition, the light source is then allowed to be sufficiently far away from the coated body and also sufficiently shielded, so that a danger of fire or explosions due ignition of the solvent containing ambient by the energetic light pulse is reduced.

According to an aspect, the THz detector comprises a THz radiation receiver and a THz optical system (e.g. one or more lenses) for directing the THz radiation having interacted with the coated body to the THz radiation receiver. The THz detector further comprises a flexible second radiation guide cable, coupling the THz radiation receiver to the laser source, so that the THz radiation receiver is enabled to receive the source laser radiation from the laser source. Thereby, the same laser source radiation received by the THz emitter can also be received by the THz detector. This allows efficient detection of the THz pulse with high temporal resolution.

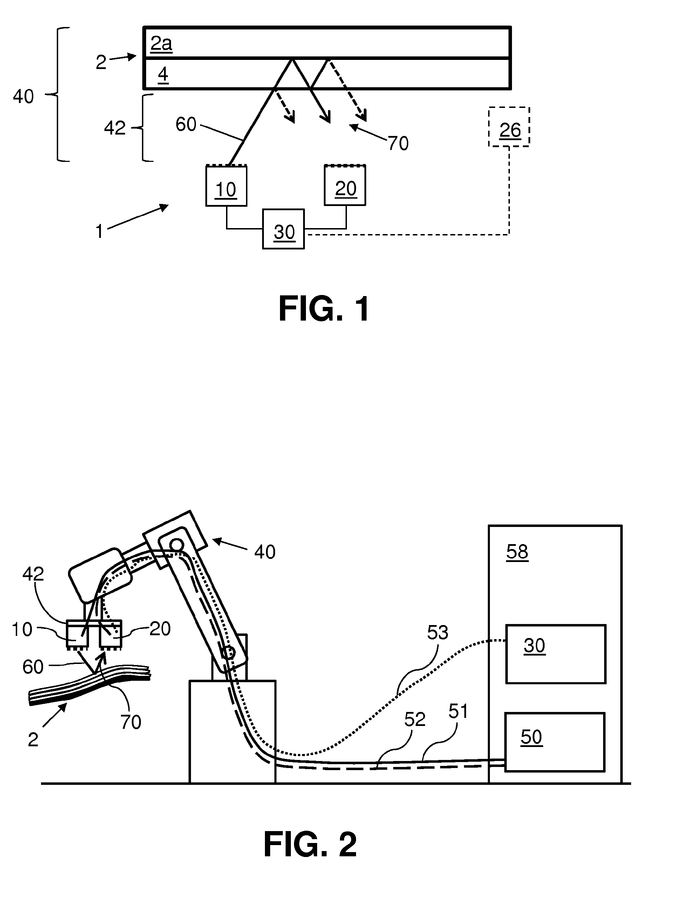

The THz detector optionally further comprises a light delaying unit adapted to delay the laser source radiation by a variable delay time, and the THz radiation receiver is coupled, in any order, to the laser source via the flexible second radiation guide cable and the light delaying unit. Thereby, the laser source radiation can be received by the THz radiation receiver in a delayed manner. This allows the THz receiver to function in an analogous manner as the THz receiver shown in Fig. 1 of EP 2 213 977 A1, with the important difference that the laser source and the THz radiation emitter / receiver are movable with respect to one another.

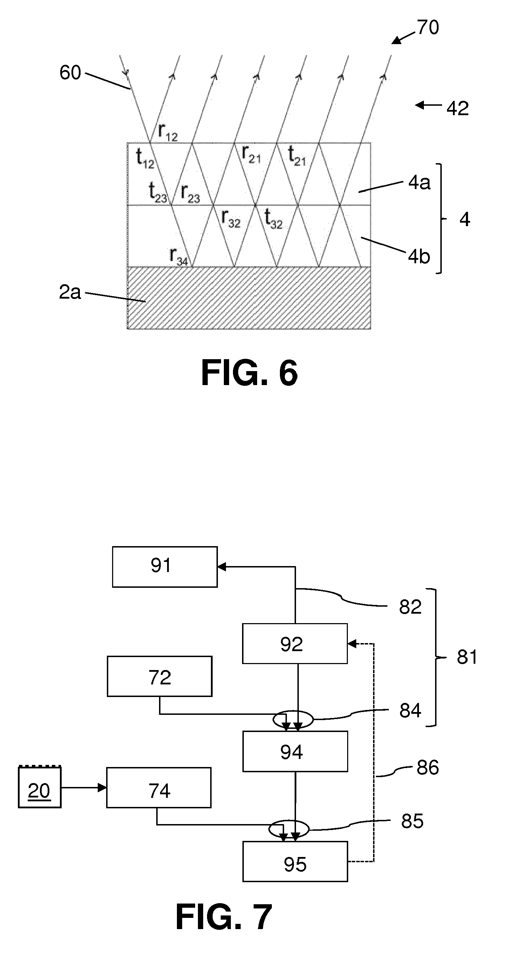

According to a further aspect, the THz radiation receiver and/or the THz radiation emitter comprise a photonic crystal (e.g. DAST, GaP, ZnTe) or a photoconductive antenna (e.g. GaAs), respectively. According to a further aspect, the THz radiation emitter/receiver is adapted for emitting/receiving the THz radiation signal as a continuous signal, a THz pulse or partial THz pulse. Herein, a partial pulse or partial wave is defined as partially - in amplitude - reflected or transmitted portions of the emitted pulse / wave: For example, each of the lines corresponding to portions of the response signal 70 in Figs. 1 and 6 indicates a partial pulse / wave.

Herein, THz radiation is defined as electromagnetic radiation of (i.e. including a non-negligible signal component having) a frequency in the range of 0.1-10 THz. The detected signal (e.g. time-domain waveform and/or frequency-domain spectrum of the detected THz radiation) is also referred to as the response signal.

Next, some aspects relating to further input data are described in more detail. According to a further aspect, the sensor system further comprises at least one of an air moisture sensor, a temperature sensor and a clock operationally coupled to the processing unit. Some or all of the sensors may be carried by the movable unit and attached to the control unit by a flexible cable such as an electrically conductive cable.

Next, some aspects relating to the geometrical arrangement of the sensor system are described in more detail. According to an aspect, the emitter system and the detector system may be arranged in a manner on the movable unit configured such that they are, in an operating state, on the same side of the coated body. This is particularly advantageous in the case that the substrate of the coated body is reflective to the THz radiation, e.g. a metal substrate of an automotive body.

Generally, it is preferred (but not required) that the emitter system and the detector system are arranged such that their lines of sight coincides. This allows the THz radiation to impinge on the coated body in a direction normal to its surface. For example, according to an aspect, the THz optical system may comprise a semitransparent THz reflector as beam splitter. The beam splitter may be arranged at an angle with respect to the coated body sheet, such that an optical path from the emitter system and an optical path to the detector system are guided to/from a common optical path that is substantially perpendicular to the coated body. As a result, the emitter system and the detector system are arranged for respectively emitting and detecting light rays having a right angle of incidence with respect to the coated body.

Other arrangements are possible as well. For example, the emitter system and the detector system can be arranged for being, in an operational state, on opposite sides of the coated body for performing a transmission measurement. This is particularly useful if the substrate of the coated body is at least partially transparent to THz radiation (e.g. transmission of at least 0.1% of the beam intensity of the THz radiation).

Next, some aspects relating to the processing of the detected response signal are described. The sensor system is configured for characterizing a coated body by any method or method steps described herein. Herein, the term "configured for" includes that the processing unit is equipped and programmed to this effect. For this purpose, a memory of the processing unit may be equipped with program code for causing a processor of the processing unit to execute the method according to any aspect described herein. According to a further aspect, the processing unit has a memory containing code therein causing the processor to perform the method steps.

According to an aspect, a method of characterizing the coated body by at least one coating parameter based on fitting to a physical model is provided. The method comprises: emitting, by the THz emitter, a THz radiation signal towards the coated body such that the THz radiation interacts with the polymeric coating; detecting, by the THz detector, a response signal being the detected THz radiation signal having interacted with the polymeric coating; determining, by the processing unit, model parameters of the physical model by optimizing the model parameters such that a predicted response signal of the physical model is fitted to the detected response signal, the model parameters being indicative of optical properties of the polymeric coating describing the interaction of the THz radiation signal with the polymeric coating; and determining, from the determined model parameters, at least one coating parameter. The at least one coating parameter may include a thickness of the polymeric coating and/or other parameters described herein.

Next, some aspects relating to the algorithm for fitting the predicted response to the detected response signal and for finding the model parameters are described in more detail. The algorithm is based on a physical model, i.e. a function outputting a predicted response signal based on model parameters as input variables.

The model parameters may include quantities of interest such as an index of refraction or a parameterization thereof. According to an aspect, the model parameters of the physical model are determined by optimizing the model parameters such that a predicted response signal of the physical model is fitted to the detected response signal. The algorithm includes the following input data: a reference waveform (in time domain) or reference spectrum (in frequency domain) or some other signal sequence describing the emitted THz radiation signal not having interacted with the coated body, and the detected response having interacted with the coated body. In addition, other parameters characterizing the coated body may be inputted, such as known properties of the coating (e.g. a known parametrization of its index of refraction), known number of layers of coating layer, known thickness of some layers if available, temperature of the coated body, etc. Likewise, other parameters characterizing the ambient medium may be inputted, such as an ambient moisture and / or a temperature. Any of these parameters can, according to a further aspect, alternatively also be obtained as input parameter which is then determined by the fitting algorithm described herein.

Preferably, an iterative algorithm is used. The iterative algorithm includes the following steps:

(a) calculating a simulated (predicted) response based on the physical model using an initial guess for the model parameters; (b) calculating an error function expressing a deviation between the predicted response and the detected response; (c) iterating steps (a) and (b), whereby instead of the initial guess in step (a) the model parameters are updated in order to reduce the error function. These steps (a) and (b) are iterated until the error function satisfies a best-fit criterion. Finally, (d) obtaining the fitted parameters as the final parameters satisfying the best-fit criterion in step (c). Then, at least some of the coating parameters (e.g. thickness) are calculated from the fitted model parameters.

The coating parameters are thus determined by calculating a best-fit response as a function of the model parameters, such that the best-fit response satisfies a predetermined best-fit criterion for an error function expressing a deviation between the predicted response and the detected response. The best-fit criterion may include a minimization criterion for the error function. The error function may include, e.g., the $L^2$ norm of the difference between the predicted response signal and the measured response signal.

Once the model parameters are determined, at least some of the coating parameters are then calculated from the model parameters.

Next, some aspects regarding the model parameters of the physical model are described in more detail. According to an aspect, the model parameters are indicative of optical properties of the coating layer describing the interaction of the THz radiation signal with the coating layer, and thereby allow calculation of a predicted response signal using the physical model. Also, once the best-fit model parameters are determined, the model parameters allow calculation of the coating parameters.

According to an aspect, the model parameters may include, for example, at least one of the index of refraction, indices of transmission and reflection, and a parameterization thereof. If multiple layer(s) of the coating are present or expected, the model parameters may include any of the parameters for each of the layers, e.g. a thickness of each layer. In addition, the model parameters may include the number of layers.

Preferably, the physical model and the model parameters enable a parameterization of the index of refraction and / or of the transmission and reflection coefficients such that these quantities have a frequency dependence (e.g. by describing at least one resonance contributing to the index of refraction). In an example, a frequency dependence can be obtained by expressing the transmission and/or reflection coefficients in terms of a frequency-dependent index of refraction of each layer. The frequency-dependent parameterization is preferably based on physical considerations. Preferably, the model parameters allow the index of refraction and / or the transmission and reflection coefficients to be expressed as complex numbers, i.e. they allow a non-zero imaginary part of these quantities.

In the following, possible model parameters for parameterizing a frequency-dependent index of refraction $n(\omega)$ of one coating layer of the coated body, $\omega$ being frequency, are given by means of example. Namely, the functional form of $n(\omega)$ may be expressed using the following parameterization that approximates the expected frequency dependence:

$$n(\omega)^2 = n_0^2 + \sum_k n_k^2 * p_k(\omega) \tag{1}$$

Herein, k=1..N is an index (N being a natural number, e.g. N=1), and $n_0$, $n_k$, are the model parameters, and $p_k(\omega)$ is a frequency dependent function that represents physical phenomena in the coating layer. The parameterization of equations has not only the advantage of approximating the expected form of an index of refraction of a coating layer well, but also allows for a physical interpretation of the frequency-dependency being caused by physically relevant modes in the coating layer, e.g. absorption modes.

According to an aspect, the processing of the detected response signal includes calculating at least one of the following coating parameters of the at least one coating layer: (a) a thickness of each coating layer and/or of of the entire coating; (b) a paint type identifier characterizing a type of paint contained in at least one layer of the coating, such as water-borne or solvent-borne paint (other possible identifiers include a type of pigments or additives). The paint type identifier is optionally obtained from, possibly among others, a parameter characterizing the frequency-dependence of the index of refraction of the respective layer; (c) a specific weight of at least one layer of the coating, wherein the weight of the layer is optionally obtained from at least one of the index of refraction and the paint type identifier of the layer; (d) a defect parameter indicating a defect in at least one layer of the coating; (e) a total number of layers of the paint film.

Next, some aspects regarding the determining of the identification of possible defects is described. According to an aspect, the model parameters may further include a parameter indicating the number of layers of the coating (e.g. as an integer-valued fitting parameter) for determining the number of layers, and / or for identifying a possible defect in coating, such as gas bubbles. The defect is detected as a jump (increase by 1) in the number of layers at a given location. This is possible because the defect interacts with the THz radiation like an additional "layer" of low index of refraction, present only at the given location. Due to the high difference in index of refraction with the surrounding coating layers, the optical contrast is high, and reliable detection of the defect is possible.

Hence, according to an aspect of the invention, a defect is detected by determining the number of layers as a function of location, and by registering a local variation in the number of layers. The defect area may then be determined as an area having an increased number of layers relative to its surrounding. Thereby, the size of the defect may be determined as the size of this area. Within this area, also the index of refraction of the defect may be determined, and therefrom optionally a type of defect may be determined.

Further details and aspects are described in the co-pending application "Sensor system and method for characterizing a coated body" of the present inventor, which has been filed on the same day at the same patent office as the present application (or at least as its priority application).

Next, some aspects relating to the characterization of a wet paint layer are described. Herein, a wet paint layer is defined as a layer that has not yet fully dried, and that still has a liquid component. According to an aspect, the model parameters and/or the coating parameters (sometimes also referred to as paint layer parameters) include a current wet layer thickness and optionally a predicted dry layer thickness of the wet layer. Namely, according to an aspect of the invention, the determining step includes determining the predicted dry layer thickness.

According to an aspect, the determining of the predicted dry layer thickness includes determining a dry-fraction parameter indicative of a relative amount of a dry portion of the wet paint layer, and determining the predicted dry layer thickness as a function of the dry-fraction parameter (which does not exclude dependence on other parameters such as the current wet layer thickness). The predicted dry layer thickness may, for example, be determined as a product of the dry-fraction parameter and the current wet layer thickness.

According to an aspect, the calculation of the dry-fraction parameter is based on the Bruggeman effective medium theory. Herein, the model parameters parametrizing the refractive index (via an effective dielectric function $\varepsilon_{eff}$ of the wet paint layer) include the dry-fraction parameter, a stored dielectric function $\varepsilon_{dry}$ of the dry component, and / or a stored dielectric function $\varepsilon_{corr}$ of a wet part of the wet paint layer.

Another aspect for determining the predicted dry layer thickness is based on a predetermined function stored in a memory of the controller, which outputs the predicted dry layer thickness as a function of prediction-relevant input parameters such as the wet layer thickness. The prediction-relevant parameters may include model parameters, other paint layer parameters, or parameters obtained from other sources such as another sensor element (e.g. temperature sensor and/or a clock). In particular, the prediction-relevant parameters comprise parameters describing at least one of the current thickness of the wet layer, the type of paint, and the elapsed time since the paint deposition. The prediction-relevant parameters may further contain at least one of the following: humidity; temperature; wet layer thickness at a first time; and wet layer thickness at a second time.

Next, some aspects relating to the method and facility for painting a body are discussed. According to an aspect, the painting facility comprises a painting unit for applying a paint layer to the body (e.g. a paint spraying unit/robot for applying a water-borne paint or a solvent-borne paint); and a sensing unit comprising the sensor system described herein. The painting facility may be a paint line of an automobile factory. The painting unit and the sensor unit may be provided in a single paint booth, which allows for immediate quality control of the paint layer. Alternatively, the painting unit and the sensor unit may be provided in different booths, which allows for quality control of the paint layer during flash-off and/or curing. According to an aspect, the painting unit is separated from the sensing unit by a distance of less than 50 m or even less than 20 m.

According to an aspect, the sensor system is adapted for characterizing the wet paint layer while the body is still being painted and/or while the wet paint layer has not yet finished the drying process. Optionally, the sensor system is operationally coupled to the painting unit for further processing the coated body in dependence of the characterized wet paint layer, e.g. of the obtained coating parameters. For example, the painting unit may be configured for adapting painting parameters in response to the coating parameters. Alternatively, the sensor system is operationally coupled to a further painting unit for further processing the coated body in dependence of the characterized wet paint layer. The further processing may include removing the painted body from the processing line temporarily (e.g. for re-painting) or permanently. The further processing may also include removing the paint and/or applying further layer(s) of paint, preferably while the wet paint layer is not yet dry.

Aspects of the invention allow quality control of painted bodies, e.g., automobile components, while they are being processed. This allows early quality control while the painted surfaces are still wet, and correspondingly early separation between correctly painted bodies and ones with defects. Due to the early separation, the process lead time can be decreased and parameters of the painting process can be adapted in short time. The sensor system and quality control method can be used for on-line, in-line, at-line and off-line quality control, but is preferred to be used in-line.

The system according to the invention is especially applicable in the case that the coating is a paint film having one or more layers of wet paint layer. One use of the system is for the analysis / painting of a painted automobile body or a painted automobile component. Another use is for the analysis / painting of a train body / component, an aircraft body / component such as an aircraft fuselage, aircraft wing, or the like. Another use is for the analysis / painting of a wind turbine component, in particular of a painted blade of a wind turbine. The substrate body may comprise at least one of a ferrous metal, a non-ferrous metal, and a fiber composite material. For example, an application of the present aspect of the invention is defect detection in blades of wind turbines e.g. for off-shore purposes. Here, the painted body is a wind turbine blade containing a defect below the wet paint layer.

Detailed description of the Figures and of embodiments:

Reference will now be made in detail to the various embodiments, one or more examples of which are illustrated in each figure. Each example is provided by way of explanation and is not meant as a limitation. For example, features illustrated or described as part of one embodiment can be used on or in conjunction with any other embodiment to yield yet a further embodiment. It is intended that the present disclosure includes such modifications and variations.

Within the following description, a paint film, possibly comprising several layers, is used as an example for the coating. It is appreciated that the teaching applies, likewise, to other coatings, in particular polymeric coatings.

Within the following description of the drawings, the same reference numbers refer to the same or to similar components. Generally, only the differences with respect to the individual embodiments are described. Unless specified otherwise, the description of a part or aspect in one embodiment applies to a corresponding part or aspect in another embodiment as well.

With reference to Figs. 1-5, embodiments of the sensor system are now described.

Fig. 1 is a schematic side view illustrating basic components and general operation of a sensor system 1 according to an embodiment of the invention. The sensor system 1 has an emitter system 10 for emitting THz radiation, a detector system 20 for detecting THz radiation, and a processing unit 30 operationally coupled to the emitter system 10 and the detector system 20. In addition, Fig. 1 shows an optional additional sensor 26, e.g. an optional humidity measurement device, thermometer, a positioning sensor and/or presence sensor, e.g. for sensing the presence and/or location of a car body. The sensor 26 may also be operationally coupled to the processing unit 30. Herein, "operationally coupled" includes an interface of the processing unit coupled to the respective system, e.g. to the emitter system for triggering emission of THz radiation and to the detector system for receiving measurement data indicative of the response signal.

Further, a painted body 2 is arranged such that the painted body 2 is faced by the emitter system 10 and the detector system 20, with an air gap 42 between the emitter and detector systems 10, 20 on the one side and the painted body 2 on the other side. The painted body 2 has a substrate 2a and a paint coating 4. In Fig. 1, the paint coating 4 has one layer. This is shown only by means of illustration, and the paint coating 4 may alternatively be a paint stack having more than one layer, e.g. two or three or four layers. According to a preferred aspect, the described method and system is available for a multi-layered paint coating having at least two layers.

Fig. 1 also shows the path of a THz radiation signal 60 emitted from the emitter system 10. The THz radiation signal 60 (solid line) traverses the air gap 42 and the painted body 2, whereupon it interacts with the painted body. A portion of the THz radiation signal, indicated by the solid line in Fig. 1, is reflected at the surface of substrate 2a and propagates back through the air gap 42 and towards the detector system 20. Other portions of the radiation signal 60, indicated by the dashed lines in Fig. 1, are partially reflected at various layer interfaces of the painted body (more precisely, they are (almost fully) reflected at the substrate side and partially reflected at the air side of the coating 4), and eventually propagate back towards the THz detector system 20 (as THz response signal 70), and are detected therein. Besides these reflections, also the propagation speed of the various portions of the THz radiation is influenced during their interaction with the painted body 2. In this manner, the detected THz signals 70 carry detailed information about the paint coating 4 of the painted body 2.

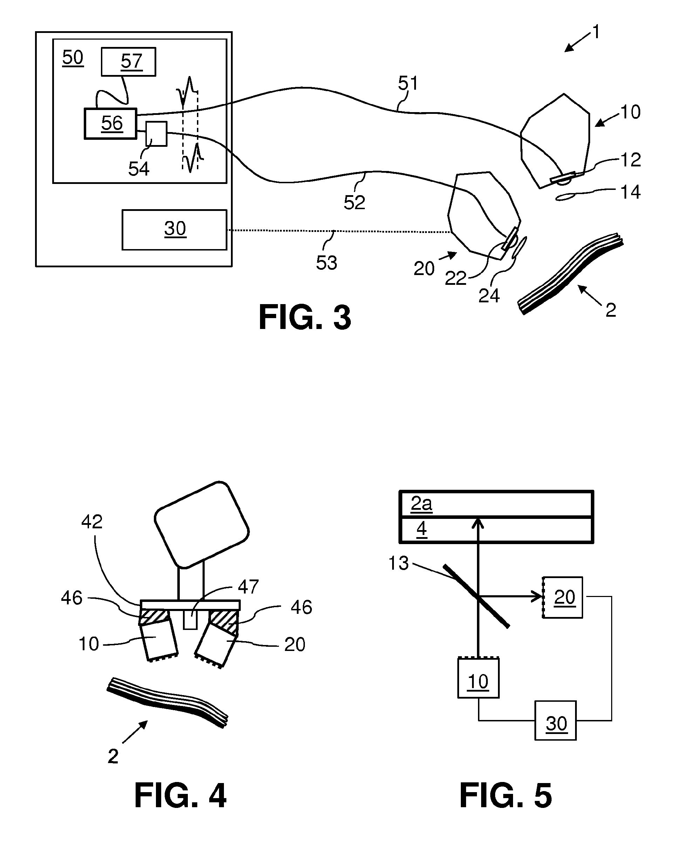

In Figs. 1 and 6, the radiation is shown to propagate along an angle with respect to the normal direction of the painted body 2. Fig. 5 is a schematic side view of further details of a possible implementation or variant of the sensor system of Fig. 1. In Fig. 5, the emitter system 10 and the detector system 20 are arranged with their axes at an angle (here: 90°), and a beam splitter 13 is arranged such as to co-align the axes. Thereby, the optical axes are co-aligned, so that the transmitted and received THz signals are collinear and normal to the surface of the painted body 2. This arrangement is especially advantageous in the case of the substrate 2a being reflective to THz radiation, e.g. in the case of a metal substrate.

Fig. 5 thus illustrates an aspect which can be combined with any aspect or embodiment herein, that the main direction of propagation of the THz radiation preferably impinges normally on the painted body so that the transmitted and received THz signals are collinear and normal to the surface of the painted body 2. In this manner, a maximum portion of the reflected signals in captured by the detector, and the reflection is minimally influenced by the geometry of the setup. Throughout the description, normal incidence is assumed, although the respective formulae can be generalized to non-normal incidence in a straightforward manner by using the Fresnel equations for non-normal incidence.

Further alternative arrangements are possible. For example, the emitter system 10 and the detector system 20 may be arranged on opposite sides of the painted body 2, for enabling a transmission measurement instead of the measurement of the embodiment of Fig. 1. This arrangement is especially advantageous in the case of the substrate 2a being at least partially transmitting THz radiation, e.g. in the case of a resin-containing substrate.

The waveform of the THz radiation response 70 is influenced by the coating's thickness and optical properties. In particular, the intensity of each partially reflected beam portion depends on the transmission and reflection coefficient(s) of the coating, and the time delay of the partially reflected beam portions with respect to the emitted beam also depends on the optical thickness of the paint layer / its layers, as described in more detail with reference to Fig. 6. Hence, the full radiation response 70, together with a reference signal corresponding to the emitted THz signal 60 not having interacted with the painted body, contains sufficient information for the determination of thickness d of the coating 4 and, if present, of the thicknesses of each of its individual layers, as well as of other paint parameters of the painted body 2.

Fig. 2 is a schematic side view of a sensor system according to an embodiment of the invention. The Figure again shows the coated body 2, and the sensor system according to an embodiment of the invention. The sensor system has a THz system with a laser source 50, a THz emitter 10, a THz detector 20, flexible radiation guide cables 51, 52 connecting the laser source 50 to the THz emitter 10 and a THz detector 20, respectively, for transmitting the source laser radiation generated by the laser source thereto. The sensor system further has a positioning system (e.g. industrial robot) 40 comprising a movable unit (robot head or teminal actuator) 42 which carryies the THz emitter 10 and the THz receiver 20.

The sensor system further has a processing unit 30. The processing unit 30 is equipped with a processor and with a memory in which software code is stored enabling the processor to carry out any method described herein. The processing unit 30 is operationally coupled, via an electrical cable 53, to the THz detector 20 for receiving and processing a detected response signal representing the detected THz radiation. The processing unit 30 may further be coupled to the emitter system 10 (via a cable not shown) for controlling the THz generator 12, e.g. electrically manipulating the THz generator by applying, for example, a bias voltage to it. Further, the emitter system may receive and processing an emitted THz signal representing the emitted THz radiation. Alternatively, the processing unit 30 may contain a memory region for storing the emitted THz signal as a pre-stored signal (e.g. from a measurement by the THz detector in which the coated body 2 is replaced by a simple THz reflector).

The positioning system 40 allows the THz emitter 10 and receiver 20 to be positioned relative to the coated body 2, by moving the movable unit 42. Thereby, the THz emitter 10 and receiver 20 are movable relative to the laser source 50 which is arranged outside of the movable unit 42. During this motion, the THz emitter 10 and receiver 20 keep being coupled to laser source 50 via the flexible radiation guide cables 51, 52.

The laser source 50 may further contain optical means to delay the laser light from the laser source 50, optical means to shorten its pulse width, optical means to further correct it for artifacts which will occur during guiding along the cable 51 (and 52), and optical means to detect the laser light. Also, while the description focuses on a laser source, also another light source can be used.

According to a general aspect illustrated in Fig. 2, the laser source 50 and the processing unit 30 are arranged in a common housing 58, which may be at least 5 m or even at least 10 m spaced apart from the THz emitter 10 and receiver 20, in at least one position of the positioning system 40. The housing 58, or at least the laser source 50, may be arranged in the same room / cubicle as the THz emitter 10 and receiver 20, or in a separate room / cubicle. With the separate arrangement, fire and explosion risk due highly energetic light pulses and/or electrical equipment near the evaporating solvents can be reduced significantly. Also, the THz components on the movable unit can be provided more compactly, which allows shielding them, and in particular the THz generator 12, more easily against inflammable gases. Hence, according to an aspect, the THz generator 12 is provided in a gas-tight housing and/or in an electromagnetically shielded housing.

The positioning system 40 can be a floor or wall mounted robot and have an arbitrary number of degrees of freedom for the movable unit 42, e.g. at least two or even all six degrees of freedom. This allows the THz emitter 10 and receiver 20 to move around the coated body 2 and to reach all of its major surfaces. The positioning system 40 is adapted for moving the movable unit 42 with at least 2 degrees of freedom, preferably with at least 3 degrees of freedom, and most preferably with 6 degrees of freedom.

The positioning system 40 allows a quality control process during which the sensor system adapts its position relative to the coated body 2 such that it scans the body and maps out the quality control parameters as a function of location, e.g. on a predefined grid. To this purpose, the robot is programmed to move in a fixed pattern along all parts of the body surface while measuring the THz radiation 70 having interacted with the surface. For the coated body being an automobile body, and for a 10x10 cm$^2$ grid size (corresponding to about 1000 points), this is possible within 5-10 seconds. 5-10 seconds is only a fraction of the time an automobile spends in the flash-off zone.

The scanning pattern itself may depend on the analysis method. Normally, the coated body 2 is scanned only once. However, the coated body 2 may be scanned twice with a predetermined time (e.g. 1-2 minutes) between two measurements at the same location, if two current wet thicknesses are desired, e.g. for predicting the dry layer thickness.

The system shown in Fig. 2 may further comprise a body positioning system (not shown) configured for positioning the coated body 2 relative to the sensor system 10 and in particular relative to the light source (50). This body positioning system may be an industrial robot holding the coated body, or a feeding mechanism of the production line.

Fig. 3 is a schematic view showing the THz system and related components of a sensor system according to an embodiment of the invention in more detail. Therein, the laser source 50 comprises a main laser 56 fed by a pumping laser 57 (if necessary). The laser source 50 is configured for generating a laser pulse capable of exciting the THz radiation 60 by impinging on the THz emitter 10, more precisely to the THz radiation generator (photoconductive antenna or photonic crystal) 12 thereof. To this purpose, the laser source 50 (more precisely, the output of the main laser 56) is coupled to THz radiation generator 12 via the flexible first radiation guide cable 51 for transmitting the source laser radiation from the laser source 50 thereto.

Besides the THz radiation generator 12, the THz emitter 10 further comprises a THz optical system 14, e.g. exemplified by a lens, for directing the outgoing THz radiation towards the coated body 2. The THz optical system 14 can comprise additional optics, e.g. lens(es) and / or mirror(s).

The THz detector 20 comprises a THz radiation receiver 22 and a THz optical system 24 for directing the THz radiation from the coated body to the THz radiation receiver 22. Again, the THz optical system 24 is exemplified by a lens, but it may comprise additional optics e.g. lens(es) and / or mirror(s). The THz radiation receiver 22 is coupled to the laser source 50 via a further flexible second radiation guide cable 52. Further, a light delaying unit 54, adapted to delay the laser source radiation by a variable delay time, is shown between the laser source 50 and the THz radiation receiver 22, so that the THz radiation receiver 22 receives the delayed source laser radiation from the laser source 50. Additional elements, not shown in Fig. 3, may be present as well, such as a pulse width shortener and other elements known to the person skilled in the art of THz emitter / detector systems.

Fig. 4 is a schematic view showing the movable unit 42 of the positioning system 40, and some components carried thereby according to an embodiment of the invention. In particular, the movable unit 42 carries the THz emitter 10 and THz detector 20.

The movable unit 42 further carries a distance sensor or positioning sensor 47 based on for instance one or more low energetic infrared light beams or based on ultrasound. The distance sensor 47 is configured for measuring the distance to the coated body. The positioning system 40 is operatively coupled to the distance sensor 47 and configured for adapting motion of the movable unit 42 such that the distance with respect to the coated body 2 is adjusted. The distance is, in particular, adjusted for focusing the THz radiation emitted / detected by the THz emitter 10 / detector 20.

The distance sensor 47 may be operable as a surface detecting unit 47 configured for detecting a surface orientation and/or surface curvature of the relevant portion of the coated body 2, e.g. by scanning the distance sensor 47 over the portion. In this case, the positioning system 40 may be adapted for motion of the movable unit 42 such that the orientation with respect to the coated body 2 is adjusted such that the THz emitter 10 and detector 20 are at a suitable angle of incidence with respect to the coated body surface 2, e.g. at normal incidence as show in Fig. 5.

Thus, the positioning system 40 may be configured for moving the movable unit 42 towards the coated body 2 while the distance (or positioning) sensor 47 measures the distance with respect to the body 2. At the distance which corresponds to the focal lengths of the THz optics 14, 24 (see Fig. 3), the positioning system 40 starts moving the movable unit 42 for scanning the automobile body in a predefined pattern, while keeping the head always at the appropriate distance (within a predefined distance band) from the surface 2 of the coated body 2. This distance is variable, but an advantageous distance is between 10 and 20 cm.

The movable unit 42 further comprises, according to an optional embodiment, an actuator system 46 for moving (e.g. rotating) the THz emitter 10 relative to the THz detector 20. A controller therof is coupled to the surface detecting unit 47, and is configured for causing the actuator system 46 to move the THz emitter 10 relative to the THz detector 20 in response to the detected surface orientation and/or surface curvature such as to direct the THz beam in a suitable manner.

With reference to Figs. 6 and 7, details of the data analysis algorithms stored in the processing unit (30) according to an embodiment are now described. The data analysis algorithms are for processing the detected response signal and for obtaining coating parameters therefrom.

Fig. 6 shows the interaction of the THz radiation with the painted body 2 in more detail, this time with the coating 4 having two layers 4a and 4b. At each interface of layers 4a, 4b - either with another layer or with the surrounding medium - a portion of the THz radiation is reflected, and a portion is transmitted. The reflected and transmitted portions are expressed by the complex reflection coefficients $r_{ij}$ and the complex transmission coefficients $t_{ij}$, respectively. Here, the indices ij indicate the boundaries between layers i and j, layer 4a being indicated by i,j=2, layer 4b by i,j=3 and the surrounding medium 42 by i,j=1. The reflection coefficient at the substrate 2 is written as $r_{34}$, i.e. in this case the index j=4 refers to the reflective substrate 2a.

The interaction of the electromagnetic radiation with this multilayer stack (air gaps 42, painted body 2 having substrate 2a and layers 4a, 4b) creates a complex pattern of reflected and transmitted signals. A portion of this THz radiation having interacted with the painted body 2 is detected by the detector system 20. This detected radiation, more precisely the set of data points representing the detected radiation (e.g. represented as a time-domain curve or as a frequency-domain curve, is also referred to as the THz response signal 70.

The interaction of light with the multilayer structure pictured in Fig. 6 can be described by the Fresnel equations. For a thin film having two layers on a metal substrate in air (refractive index $n_1$=1), the first layer having refractive index $n_2$, thickness $d_2$ and the second layer having refractive index $n_3$, thickness $d_3$, the reflected total electric field $E_r$ can be written as a series of the partial rays:

$$E_r = E_0 \left( r_{12} + t_{12}r_{23}t_{21}e^{-i2\beta} + t_{12}r_{23}r_{21}r_{23}t_{21}e^{-i4\beta} + \ldots \right.$$
$$\left. \ldots + t_{12}t_{23}r_{34}t_{32}t_{21}e^{-i2\gamma} + t_{12}r_{23}r_{21}r_{23}r_{21}r_{23}t_{21}e^{-i6\beta} + \ldots \right) \tag{2}$$

Herein, assuming normal incidence of the radiation, the indices of transmission and reflection $t_{ij}$ and $r_{ij}$ and the phase shifts $\beta$ and $\gamma$ can be expressed as follows:

$$t_{ij} = \frac{2n_i}{n_i + n_j} \qquad r_{ij} = \frac{n_i - n_j}{n_i + n_j} \tag{3}$$

$$\beta = \frac{2\pi}{\lambda}d_2n_2 \qquad \gamma = \frac{2\pi}{\lambda}(d_2n_2 + d_3n_3)$$

with $\lambda$ the wavelength of the incident light, and $n_i$ being the (complex and possibly frequency-dependent) index of refraction and $d_i$ the thickness of the respective i-th layer (or air or the substrate) as described above.

The index of refraction for the paint layer is expressed in a particular manner which takes into account the particular characteristics of the paint layer. More details about the parametrization of the paint layer's index of refraction are discussed further below.

The processing section 30 (see Fig. 1) receives the response waveform (THz radiation response) 70, and also receives, or has stored therein, the waveform 60 emitted by the emitter 10. The processing section 30 then performs an analysis of the response waveform (taking into account the original waveform and other information such as detected moisture and/or temperature), and thereby obtains the paint parameters by the method described herein (see e.g. the description of Fig. 7 for additional details).

In the following, specific aspects of the iterative algorithm for obtaining thickness of the paint and other paint parameters are described. The inventors have found that a stable and reliable algorithm is obtained by determining the paint parameters using a physical model. Here, the paint parameters include at least one thickness of the paint layer of the painted body, e.g. the total thickness of the paint and/or the paint layer of one or more of its sub-layer(s). For definiteness, the method is illustrated for the case of a substrate 2a on which a paint consisting of two layers 4a, 4b is arranged (see Fig. 1), and for the following paint parameters to be determined: thicknesses d2, d3 of each of the layers (the thicknesses are collectively labeled as d); and other paint parameters that can be expressed in terms of the frequency-dependent index of refraction $n(\omega)$ of each layer. The discussion herein can be adapted to the case of determining a thickness of a single layer of the paint or to the thicknesses of each of more than two layers.

This algorithm is illustrated in the block diagram of Fig. 7 in more detail. This algorithm is based on a physical (optical) model 81. The physical model 81 includes a waveform-prediction map 84 that maps the model parameters 92 as input to a predicted waveform 94 as output. Further, the model 81 includes a paint-parameter map 82 that maps the model parameters 92 as input to the paint parameters 91 as output. Herein, the model parameters 92 are, for example, a parameterization of the index of refraction $n(\omega)$ and the thickness d for each layer; and the predicted waveform 94 is, for example, a predicted form of the response signal 70.

In the following, an example of the waveform-prediction map 84 is described in more detail. As stated above, the waveform-prediction map 84 takes the model parameters 92 as input and outputs a predicted waveform 94. Here, the model parameters are the thickness d for each layer (i.e. in the example thicknesses d2, d3), and a parameterization of the frequency-dependent index of refraction $n(\omega)$ for each layer.

In the following, preferred aspects of the parameterization of the index of refraction $n(\omega)$ are described that can be used independently of the given embodiment. According to a first aspect, a general parametrization of the index of refraction is described. The inventors surprisingly found out that this parameterization is sufficiently general so that it also allows reliable results in the case of a paint layer, and not only of a dry paint layer. A main reason is that the paint layer can be treated as a homogenous medium on the length scales of the THz wavelength.

The parametrization of the index of refraction $n(\omega)$ is such that the index of refraction has a dependence on frequency, wherein the index of refraction preferably has the form of Eq. (1) above. Preferably, the index of refraction includes a frequency-dependent contribution describing a resonance, and the frequency-dependent contribution is particularly preferably expressable as a function $p_k(\omega)$ proportional to

$$\omega_{p,1}{}^2 / \left(\omega_0{}^2 - \omega^2 - i\gamma_1\omega\right),$$

wherein $\omega$ is the frequency, $\omega_0$ is an oscillator frequency, $\omega_{p,1}$ is a plasma frequency, $\gamma_1$ is a damping coefficient, and i is the imaginary unit. Alternatively or additionally, a frequency-dependent contribution may be expressable as a function $p_k(\omega)$ proportional to

$$\omega_{p,1}{}^2 / \left(-\omega^2 - i\gamma_1\omega\right),$$

i.e. as a free oscillator having a peak at zero frequency.

Optionally there are other frequency-dependent contributions / summands, e.g. contributions from other oscillators.

For example, a possible parameterization of the (squared) index of refraction is

$$n(\omega)^2 = \epsilon(\omega) = \epsilon_\infty + \sum_{i=1}^{n} \frac{\omega_{p,i}^2}{\omega_{0,i}^2 - \omega^2 - i\gamma_i\,\omega} \tag{4}$$

where $\epsilon_\infty$ is the dielectric constant at high frequencies, and $\omega_0$ the oscillator frequency. A paint layer, as far as its interaction with THz radiation is concerned, can be expressed, for example, by one or two oscillators: One free oscillator (for which $\omega_0 = 0$); and optionally another oscillator being associated with an absorption band (for which $\omega_0$ has some finite value).

In the case of multiple layers, the parameterization as described above can be used for each of the layers including the wet layer. Hence, the model parameters in this case are the adjustable parameters in Eq. (4) and the thickness d, for each of the layers. Thus, for example, in the case of layers each being modelled by just one oscillator representing the free electron oscillations of the layer (i.e. with $\omega_0 = 0$), the model parameters for each layer are d, $\varepsilon_\infty$, $\omega_{p,1}$ and $\gamma_1$, and the index of refraction is obtained via Eq. (4) with counter n=1 and $\omega_{0,1}^2 = 0$.

According to a second aspect of the parametrization, the index of refraction n is provided as a function $n(\omega,f)$ of the frequency $\omega$, wherein this function further depends on the dry-fraction f (and possibly on further parameters). Herein, f is used as a model parameter, and the best-fit of f is obtained as for the other model parameters. This has the advantage that the parameter (dry-fraction) f can be used for predicting the dry-layer thickness.

According to an aspect, the functional form of $n(\omega,f)$ may depend of a determined or preconfigured type of paint. In a particular embodiment, the type of paint may be used as a further (discrete) parameter for which the best fit is obtained.

According to a further aspect, the function $n(\omega,f)$ may be based on the Bruggeman approximation described further below.

From the thickness d and such a parameterization of the index of refraction $n(\omega)$, the transmission and/or reflection coefficients can be obtained via Fresnel equations. In the example of the painted body 2 shown in Fig. 6, the reflection and transmission coefficients $r_{ij}$, $t_{ij}$ at the interfaces of the layers 4a, 4b are, for example, given in Eq. (2-3) above.

The waveform-prediction map 84 further includes a set of optics equations for calculating a predicted response (predicted waveform for the response signal 70) 94. These optics equations may, for example, be expressed by Eq. (2) above. The optics equations have the following input parameters: (i) the waveform $E_0$ of the emitted THz radiation signal 70 (i.e. waveform of emitted radiation 60 of Figs. 1 and 3), and (ii) the reflection and transmission coefficients ($r_{ij}$, $t_{ij}$) and the phase shifts $\beta$, $\gamma$ from Eq. (3). Other input parameters may be included as well.

The algorithm further includes an error function 85 that expresses a deviation 95 between the predicted response 94 on the one hand and the detected response 74 (waveform of the detected radiation 70 of Figs. 1, 3) on the other hand. This error function 85 may, for example, be the $L^2$ norm or some other norm as described herein.

Possibly, according to a general aspect of the error function independently of this embodiment, the error function may, include a "penalty term" that penalizes a physically implausible predicted response; and/or a frequency-dependent term that gives additional weight to deviations in a particularly sensitive frequency range. Such a sensitive frequency range may include the frequency range between 0.1 THz and 1 THz at least partially. Such a term may, for example, be added to other contributions such as the $L^2$ norm.

According to a particular aspect, the error function has a frequency dependent sensitivity. Hence, a particular difference between the frequency-domain predicted response signal and the frequency-domain measured response signal may lead to an error function whose magnitude depends on the frequency at which the difference occurs.

Next, the paint-parameter map 82 is described in more detail. As stated above, the paint-parameter map 82 calculates, from the model parameters 92, the paint parameters 91 as output.

In the example parametrization of n described above, some paint parameters of the painted body may be obtained from the above parameterization of $n(\omega)$ as follows:

(a) A paint type identifier characterizing a type of paint may be determined from the parameters parametrizing $n(\omega)$, e.g. the parameters on the right side of Eq. (4). These values are then matched to a table in which the values or ranges of these parameters for each paint type are defined, and the paint type is determined based on the matching. Alternatively, only a set of discrete parameters parametrizing $n(\omega)$ may be used as input parameters of the fitting algorithm, each set of parameters corresponding to a preconfigured paint type. The set minimizing the error function is then used, and the paint type is determined as the paint type corresponding to the chosen set.

(b) A specific weight of paint layer may be directly derived from the paint type identifier of the layer, or may be obtained in a manner analogous to the method discussed in (a) above. Alternatively, for some paint types the specific weight may be expressed as a function or functional of the index of refraction, e.g. its value at a particular frequency (such as $\omega=0$) or its integral or L2 norm over a frequency range. The function or functional may also depend on the paint type described above.

(c) a defect parameter indicating a defect in the paint layer.

(d) the predicted dry thickness of the wet layer (further details discussed in separate section herein)

The wet layer thickness d was already used as a fit parameter and is identically used as paint parameter.

Likewise, the number of layers N may be used as a (discrete) fitting parameter which is then identically used as a paint parameter.

Next, the iterative algorithm itself, as illustrated in Fig. 7, is described in more detail. In a first step, initial fit parameters 92 are generated, e.g. as random numbers or plausible initial values. In this example, as stated above, the fit parameters are given by the respective thickness and parameters characterizing the respective index of refraction of each layer.

Then, the initial fit parameters 92 are input, together with the reference waveform 72, into the waveform-prediction map 84; and the waveform-prediction map 84 calculates the predicted (simulated) response 94 using this input. Namely, the indices of refraction and transmission and phase shifts are calculated via the Fresnel equations, Eq. (2-3), and the predicted response 94 is calculated based on these coefficients using the optics equations, Eq. (2), as described above.

Then, the deviation 95 between the predicted response 94 and the measured response 74 is calculated using the error function 85. Then the model parameters 92 are varied depending on the coefficients and error function 85 of previous steps. This variation is performed using a strategy that eventually approaches a minimum deviation. For example, a minimization algorithm based on the Levenberg-Marquardt technique can be used. Then, the algorithm is repeated (arrow 86), now using the varied model parameters 92 instead of the initial parameters.

In this manner, the model parameters (fit parameters) 92 are varied repeatedly in the loop represented by the arrow 86, until the deviation 95 satisfies a best-fit criterion (e.g. until the deviation is sufficiently minimized or until some other cut-off criterion is met).

Then, the final fit parameters 92 of the last step are used for calculating the paint parameters 91 (e.g. thicknesses $d_2$, $d_3$) via the paint-parameter map 82 as described above.

In this manner, the paint parameters 91 are determined by calculating a best-fit response 94 that sufficiently minimizes the deviation 95, i.e. such that the predicted response 94 of the physical model fits to the detected response 74. Since the algorithm takes into account the full waveform of the detected response 74 via the error function 85, and not just individual landmark features, the result is stable and reliable by the fact that one accounts for each individual frequency component in the appropriate way, given by the physical model.

In alternative embodiments, the frequency-dependent index of refraction $n(\omega)$ may alternatively also be replaced by another equivalent parameterization, e.g. the conductivity which is proportional to the index of refraction squared multiplied by frequency. Alternatively, also some other parameterization of the optically relevant properties of each layer can be used as fit parameters. For example, in a variation, the paint parameters 91 can be used directly as fit parameters. In another variation, the iterative method can be adapted to more than two layers. To this purpose, Eq (3) is generalised to more than 2 layers, which is straightforward textbook knowledge. In another variation, additional input parameters may be used (e.g. the index of refraction of the surrounding medium, e.g. air, 42, 44).

In another variation, some parameters described as fitting parameters may be determined using additional sensors or input means. Thus, for example the thickness d2 of the first paint layer 4a may be manually input, and the iterative method described herein may be used only for obtaining the thickness d3 of an additionally applied layer 4b.

Next, the determining of the predicted dry layer thickness is described. This paint parameter allows reliably predicting the dry thickness the wet layer will have after drying (e.g. evaporation and/or curing), when the THz measurement of the wet layer is performed at an arbitrary moment in any wet state between fully wet and fully dry.

An important general aspect of all states of paint during drying is that, when probed with THz radiation, the wavelength of the radiation is always larger than the smallest domain size. For this reason, the wet paint in each stage can be considered as being homogeneous. The inventors have found that for this reason the wet paint layer can be considered as an effective homogenous medium. This allows using the methods described herein.

According to a particular physical model that can be used with any embodiment described herein, the refractive index (more precisely, the dielectric function $\varepsilon$eff) of the wet layer is described by means of an effective medium theory in the Bruggeman approximation. Within this approximation, the dielectric function, and thereby $\varepsilon$eff, is obtained from the THz data by the best-fit algorithm described above. To this purpose, within the Bruggeman approximation $\varepsilon$eff (and thereby the refractive index) is parametrized by the dry-volume fraction parameter f:

$$f\,\frac{\varepsilon_{\mathrm{dry}} - \varepsilon_{\mathrm{eff}}}{\varepsilon_{\mathrm{dry}} + 2\varepsilon_{\mathrm{eff}}} + (1 - f)\,\frac{\varepsilon_{\mathrm{corr}} - \varepsilon_{\mathrm{eff}}}{\varepsilon_{\mathrm{corr}} + 2\varepsilon_{\mathrm{eff}}} = 0 \tag{5}$$

Here, $\varepsilon$dry is the frequency dependent dielectric function in the dry state, $\varepsilon$eff is the present frequency dependent dielectric function of the wet film, f is the dry volume fraction (0 < f < 1), and εcorr is a frequency dependent dielectric function which represents the optical difference between the fully wet state and the fully dry state but which is independent of f. The physical considerations underlying Eq. (5) are as follows: In the wet state, the paint layer has optically to be seen as being composed of a host material with inclusions of dry material. The volume fraction of the latter is nonzero and well below 1. With increasing drying time, the inclusions increase in volume fraction and eventually when the paint is fully dry, they determine the entire system (f = 1). Any state in between the fully wet and fully dry state can be described by the above equation with 0 < f < 1.

The above equation (5) allows obtaining εeff (e.g. numerically) as a function of f when the other parameters are known. For the present algorithm, εeff is obtained from the THz data, and the parameters εdry, εcorr may be retrieved as paint-specific data from a memory of the processing unit in dependence of the known paint type.

The paint-specific parameters εdry, εcorr can be obtained by a previous calibration measurement for the type of paint: εdry is obtained by measuring the dried paint's refractive index, and εcorr can be obtained by measuring various wet paint's effective refractive index (more precisely εeff) at various stages of drying, i.e. for various known values of f, and then solving the above equation (5) for εcorr of this paint type. The respective values of f are obtained from thickness measurements during the time of measuring εeff and by using the below Eq. (6). Then, the parameters εdry, εcorr for this paint type are stored in memory to be retrieved later as described above.

Thus, the dry-volume fraction parameter f is available as a model parameter which parametrizes the refractive index as described above via Eq. (5). The value of f, as well as the value of the other model parameters such as the wet layer thickness d, is then determined from the THz measurement by the best-fit algorithm described herein.

The predicted thickness of the dry film ddry is simply given by the product of the former two,

$$d_{dry} = f \times d$$

(6)

This method can be carried out at any time in the drying process, and the time between finishing the paint deposition and the measurement does not need to be known. The method works not only for a single wet paint layer directly on a substrate, but also for a wet paint layer on top of one or multiple dry paint layers.

As an alternative to the Bruggeman approximation, the predicted dry thickness of a wet multilayer paint may also be determined using stored information of the drying process. Namely, the drying behavior of each specific kind of paint as a function of a variable, which can include time (herein always understood as elapsed time after paint deposition) and/or temperature and/or humidity, is known and may be stored and used as calibration data. The calibration data can for instance be the wet thickness as a function of time at a given humidity and temperature.

Thus, the wet thickness of an individual wet paint layer is obtained from the THz response signal by fitting to a physical model as described above. Given the elapsed time between the paint deposition and the measurement of the wet thickness, and optionally other parameters such as temperature and/or humidity, the dry thickness ddry can be obtained from the stored calibration curve(s).

In the various methods for predicting ddry discussed herein, a dependence on additional parameters such as paint type, temperature and/or humidity may be omitted or partially omitted in automatized processes in which all or some conditions such as the exact composition of wet paint layer, the state of the paint before deposition, temperature, humidity etc. are kept constant.

The methods for predicting ddry discussed herein are remarkably reliable. Previously, in the absence of the THz measurement and data analysis described herein, it would have been believed that the behaviour of paint is too complex for predicting ddry reliably based on the limited available data. This is also because, depending on the kind of wet paint layer, many different processes may occur during the drying. Among these are chemical reactions between constituents, simple evaporation and cross-linking processes (polymerization). These processes were believed to each require a very sophisticated model in order to predict the dry state thickness. In contrast, by identifying models that captures the essential aspects of the drying process, as well as by using a method that obtains sufficient data of the paint, these difficulties could be overcome.

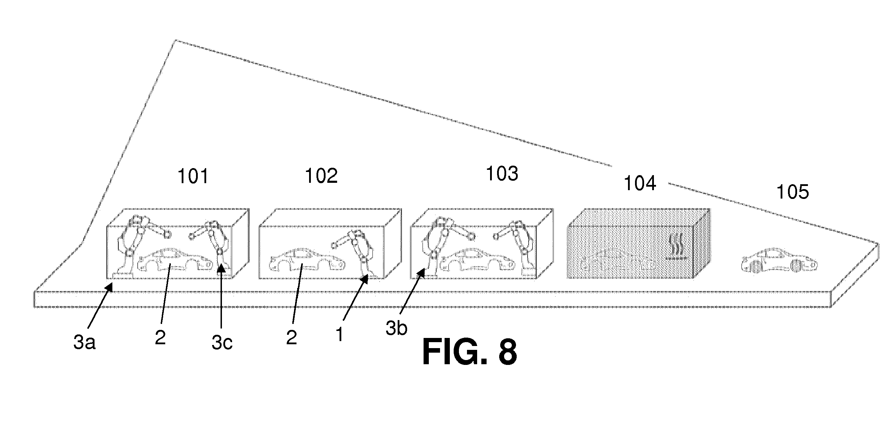

Next, a paint system and a painting process using the system according to the invention are described with reference to Fig. 8. In the following, the painting of an automobile is described as an example, but the example can be generalized to the coating of other bodies.

Fig. 8 shows a schematic drawing of the paint system being a paint line. The paint line has a number of cubicles for e.g. painting, flash-off, quality control, readaptation of the painting and curing, namely a paint booth 101, a cubicle (flash-off zone) 102 for quality control of paint based on THz technology, an (optional) further paint booth 103 for correcting the paint layers, a heated cubicle (furnace) 104 for curing the paint, and an exit 105 towards the next processing step.

The paint system may include further: A transportation mechanism for transporting the painted body 2 from the paint booth 101 through the other cubicles towards the exit 105; climate control in each cubicle; a temperature and humidity sensor in each cubicle; robots which are equipped for at least one of painting the automobile body; being sensor systems 1 for performing quality control of the painted bodies; or handling robot(s) for carrying the painted bodies.

Next, the individual cubicles and their functionality in the paint system of Fig. 8 are described in more detail. The paint booth 101 has a painting unit (painting robot) 3 a for applying a paint layer to the body 2. Optionally, more than one paint layer may be applied. A further robot 3b is provided for handling the automobile component, e.g. moving it for being painted properly. Subsequently the transportations system moves the body to the flash-off zone 102, where early quality control based on THz technology is performed.

The flash-off zone 102 has a sensor system 1 according to the invention for quality control right after the paint deposition, preferably while the paint is still wet. Thereby, an early observation of possible defects on the painted surface is possible. As described above, the sensor system 1 is configured to scan the automobile body with a predefined pattern, such as to obtain quality parameters, such as at least one of the thickness of the wet paint and a prediction of the dry state thickness and information about other possible defects. This information may be mapped onto the entire scanned automobile body surface. Thus, the sensor system 1 enables non-contact and non-destructive early quality control of the freshly deposited paint layers on automobile bodies while being processed in the paint line.

If a fault is sensed by the sensor system 1, the automobile body 2 can be removed from the main line at an early stage, such that it is ensured that the downstream line only contains bodies which are correctly painted. Moreover, by providing the sensor system 1 in the flash-off zone, where the body anyway has to wait for the solvent to partially evaporate, the quality control does not take up any extra time and on the contrary strongly reduces the lead time of the correctly painted bodies by enhancing the efficiency of the main paint line.

Optionally, the automobile body 2 may undergo an additional corrective painting step either in the flash-off zone 102 or, after being transported back, in the paint cubicle 101, or in an optional further paint cubicle 103 (by further painting robot 3b). The latter option allows the body 2 to stay in the main line.

The painting process typically involves two to three layers. These layers can be deposited all in one paint booth 101 (wet-on-wet technique), or there can be additional paint booths (not shown) and associated cubicles for each additional layer, either after a flash-off cubicle 102 or after a curing furnace 104. Quality control by the sensor system 1 can take place after each paint booth or cubicle or only after a specific one.

Optionally, the paint system may have a close loop feedback control system which receives data from the sensor system 1 in cubicle 102 and sends it directly or indirectly to prior equipment in the process line, such as the paint robot 3a in cubicle 101. An indirect sending would be provided if the data is sent via another entity which has capability other than mere forwarding of the data, e.g. via a processing unit which calculates the adapted program for the robot. The close loop feedback system influences the process parameters of the paint robot 3a depending on the data received from the sensor system 1. Alternatively or additionally, the feedback control system may send the data also to later equipment in the process line, such as to paint robot 3b in cubicle 103. The close loop feedback system then influences the process parameters of the paint robot 3b depending on the data received from the sensor system 1.

Thus, the close loop feedback control system can be used in the case that the deviations of the quality parameters resulting from the early quality control are for instance reproducible for several painted bodies 2 and/or seem to be systematic. In these cases the systematic issues can be corrected in a timely manner.

Further alternatives and extensions to the embodiments described herein are possible. Extensions can, for instance, be provided by adding equipment to the system after the early quality control which deals with the consequence of the (negative) outcome.

Fig. 8 suggests an in-line paint booth which immediately corrects the failures of the paint layers in cubicle 103. However, there may also be an alternative route in the system (horizontal but also vertical) which leads the painted body 2 to another zone of the factory and / or which removes the particular painted body from the product line. From such an alternative route, again two options exist: either leaving the corrected automobile bodies on a separate process line, or reentering them onto the main line of Fig. 8.

Although the Invention has been mainly described for early quality control of just deposited paint layers which are still wet, its use is not restricted to this application, and the quality control can for instance also and/or additionally be placed after the heated furnace 104 in order to perform quality control of dried paint layer(s). Also, the invention can be adapted to other painted bodies, such as not only an automobile component, but also a train component, an aircraft component, and a wind turbine component, and others. Thus, while the foregoing is directed to embodiments, other and further embodiments may be devised without departing from the basic scope determined by the claims.

**Claims**

**1.** Sensor system for characterizing a coating (4), such as a paint film, of a coated body (2) in a non-contact manner by use of THz radiation, the sensor system comprising a THz system (1), a processing unit (30) and a positioning system (40), wherein:

the THz system (1) comprises

- a light source (50) for generating a source light radiation;
- a flexible first radiation guide cable (51) for transmitting the source light radiation;
- a THz emitter (10) comprising a THz radiation generator (12) coupled to the laser source (50) via the flexible first radiation guide cable (51) for receiving the source light radiation from the light source (50) and adapted for generating outgoing THz radiation (60) from the source light radiation, and a THz optical system (14) for directing the outgoing THz radiation towards the coated body (2); and
- a THz detector (20) for detecting incoming THz radiation (70) having interacted with the coating (4), and wherein

the positioning system (40) comprises a movable unit (42) carrying the THz emitter (10) so that by moving the movable unit (42) the THz emitter (10) is positioned relative to the coated body (2), and wherein the light source (50) is arranged outside of the movable unit (42) so that when the movable unit (42) is moved, the THz emitter (10) is moved relative to the light source (50) while the THz emitter (10) keeps being coupled to light source (50) via the flexible first radiation guide cable (51), and wherein the processing unit (30) is operationally coupled to the THz detector (20) for receiving and processing a detected response signal representing the detected THz radiation.

2. The sensor system according to the preceding claim, wherein movable unit (42) further carries the THz detector (20) so that by moving the movable unit (42) the THz emitter (10) and the THz detector (20) are positioned relative to the coated body (2).

3. The sensor system according to the preceding claim, further comprising a flexible second radiation guide cable (52) and a light delaying unit (54) adapted to delay the light source radiation by a variable delay time, wherein the THz detector (20) comprises a THz radiation receiver (22) and a THz optical system (24) for directing the THz radiation from the coated body to the THz radiation receiver (22), wherein the THz radiation receiver (22) is coupled to the light source (50) via the flexible second radiation guide cable (52) and the light delaying unit (54) for receiving the delayed source light radiation from the light source (50).

4. The sensor system according to any one the preceding claims, wherein the positioning system (40) is adapted for moving the movable unit (42) with at least 2 degrees of freedom, preferably with at least 3 degrees of freedom, and most preferably with 6 degrees of freedom.

5. The sensor system according to any one of the preceding claims, further comprising an actuator system (46) for moving the THz emitter (10) relative to the THz detector (20).

6. The sensor system according to the preceding claim, further comprising

- a surface detecting unit (47) configured for detecting a surface orientation and/or surface curvature of a portion of the coated body (2) facing the THz emitter (10) and/or the THz detector (20), wherein the actuator system (46) is configured to move the THz emitter (10) relative to the THz detector (20) in response to the detected surface orientation and/or surface curvature.

7. The sensor system according to any one of the preceding claims, wherein the processing unit (30) is further operationally coupled to the emitter system (10) for controlling the THz generator (12).

8. The sensor system according to any one of the preceding claims, wherein the coated body (2) is one of an automobile component, a train component, an aircraft component, and a wind turbine component, and wherein the coated body (2) comprises at least one of a ferrous metal, a non-ferrous metal, and a fiber composite material as a substrate, and wherein the coating (4) is a paint film, preferably having at least one of the following layers:

(a) An e-coat layer
(b) A primer layer

(c) A base coat layer

(d) A clear coat layer.

or a combination of (a)-(d).

9.  The sensor system according to the preceding claim, wherein the processing of the detected response signal includes calculating at least one of the following coating parameters of the paint film (4) and/or, if present, of at least one of the first and second coating layers (2a, 2b):

    (a) a thickness;

    (b) a paint type identifier characterizing a type of paint contained in at least one layer (4a) of the coating (4), such as water-borne or solvent-borne paint;

    (c) a specific weight of at least one layer (4a) of the coating (4), wherein the weight of the layer is optionally obtained from at least one of the index of refraction and the paint type identifier of the layer;

    (d) a defect parameter indicating a defect in at least one layer (4a) of the coating (4);

    (e) a total number of layers of the paint film (4).

10. The sensor system according to any one of the preceding claims, wherein the coating includes a wet paint layer having not yet finished a drying process during which the wet paint layer becomes a dry paint layer, and wherein the processing of the detected response signal includes calculating a predicted dry layer thickness of the wet paint layer.

11. The sensor system according to any one of the preceding claims, wherein the processing unit (30) is adapted for characterizing the coating (4) by a plurality of coating parameters based on fitting to a physical model, and wherein the processing unit comprises a processor and a memory containing code therein causing the processor to perform the following steps:

    - Determining model parameters of a physical model by optimizing model parameters such that a predicted response signal of the physical model is fitted to the detected response signal, the model parameters being indicative of optical properties of the coating (4) describing the interaction of the THz radiation signal with the coating; and

    - Determining, from the determined model parameters, the coating parameters.

12. Painting facility for painting a body, the painting facility comprising

    - A painting unit for applying a paint layer to the body; and

    - The sensor system according to any one of the preceding claims, wherein the sensor system is adapted for characterizing the coating (4) including the applied paint layer in a non-contact manner by use of the THz radiation.

13. The painting facility according to claim 12, wherein the sensor system is adapted for characterizing the coating (4) including the applied paint layer while the paint layer is a wet paint layer having not yet finished a drying process during which the wet paint layer becomes a dry paint layer.

14. The painting facility according to claim 13, wherein the sensor system is operationally coupled to the painting unit or to a further painting unit for further processing the coated body (2) in dependence of the characterized wet paint layer, while the wet paint layer has not yet finished the drying process.

15. The painting facility according to any one of claims 12-14, further comprising a body positioning system configured for positioning the coated body 2 relative to the sensor system (10) including to the light source (50).

**FIG. 1**

**FIG. 2**

**FIG. 3**

**FIG. 4**

**FIG. 5**

**FIG. 6**

**FIG. 7**

FIG. 8

Europäisches
Patentamt

European
Patent Office

Office européen
des brevets

## EUROPEAN SEARCH REPORT

Application Number

EP 14 15 2844

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2013 228330 A (JFE STEEL CORP) 7 November 2013 (2013-11-07) * paragraphs [0002] and [0041], figures 17a, 17b, 18a, 18b * | 1-15 | INV. G01B15/02 G01B11/06 |
| A | TETSUO IWATA ET AL: "Prediction of the Thickness of a Thin Paint Film by Applying a Modified Partial-Least-Squares-1 Method to Data Obtained in Terahertz Reflectometry", J INFRARED MILLI TERAHZ WAVES,, 14 August 2013 (2013-08-14), pages 646-659, XP002722028, DOI: 10.1007/S 10762-013-0015-2 * Chapter 3 Numerical Simulations; Chapter 4.2 Wet Paint Film * | 1-15 | |

TECHNICAL FIELDS
SEARCHED      (IPC)

G01B

The present search report has been drawn up for all claims

| Place of search | Date of completion of the search | Examiner |
|---|---|---|
| Munich | 25 March 2014 | Malcoci, Andrei |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 14 15 2844

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2014

| Patent document cited in search report | Publication date | Patent family member(s) | Publication date |
|---|---|---|---|
| JP 2013228330 A | 07-11-2013 | NONE | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6484121 B1
- JP 2004028618 A
- EP 2213977 A1